# EUROPEAN PATENT APPLICATION

(11) **EP 4 545 963 A1**
(43) Date of publication of application: **30.04.2025**
(21) Application number: 23205985.7
(22) Date of filing: 26.10.2023
(51) Int. Cl.: G01N 33/50, A61K 39/00, G01N 21/01, G01N 21/64, G01N 33/574, G01N 33/543, G01N 33/58

(54) **METHOD FOR SELECTING ANTIGEN RECOGNIZING MOIETIES HAVING SPECIFICITY FOR A TARGET STRUCTURE**

(71) Applicant: Miltenyi Biotec B.V. & Co. KG, 51429 Bergisch Gladbach (DE)
(72) Inventor: BETHKE, Maria, 51429 Bergisch Gladbach (DE); HERBEL, Christoph, 51429 Bergisch Gladbach (DE)
(74) Representative: Biervert, Christian

(57) **Abstract**

The present invention provides an in-vitro method that allows to assess specificity as well as tissue cross reactivity of novel binders, i.e. antigen recognizing moieties such as antibodies or antigen binding fragments thereof, wherein said binders may be beneficially used in e.g. CAR T cell therapy.

## Description

### Field of the invention

The present invention generally relates to the field of methods for identifying an antigen recognizing moiety having specificity for a target structure, e.g. a target antigen in a specific tissue, the invention relates in particular to field of a method for identifying an antigen recognizing moiety having specificity for a target antigen in a specific tissue, and wherein said antigen recognizing moiety show low tissue cross reactivity.

### Background of the invention

Efficacy is an important feature of a drug. However, safety of a drug is at least as important. Antibodies or antigen binding fragments thereof are critical components of many successful and efficient immune therapeutic approaches to treat various diseases.

One part of the success of antibodies or their antigen binding fragments is the intrinsic specificity to their cognate epitopes. Unfortunately, cross reactivity, i.e. binding of an antibody or antigen binding fragment thereof to an unintended epitope or antigen, is an unwanted effect.

As lack of specificity can directly affect the safety of a drug, it is of high importance to characterize antibodies or antigen binding fragments thereof during drug development. One important example where lack of specificity of an antigen binding fragment of an antibody can directly affect the safety of an immunotherapy is when the drug is a chimeric antigen receptor (CAR) for use in CAR immune cell therapy.

One prominent way to assess binding properties of antibodies or antigen binding fragments thereof before *in vivo* application is histological staining, which can be laborious and time consuming.

For the development of novel antibodies and antibody-derived fragments for therapeutic approaches it is important that new antibodies or fragments thereof do not bind to structures other than the targeted epitope.

One approach to characterize the binding specificity of a new antibody or fragment thereof is a tissue cross reactivity study. The goal is safety assessment by identification of cross-reactive binding, i.e. on different diseased, malignant, or healthy tissues, and off-target binding across a range of tissues. A tissue cross reactivity study is a screening assay utilizing immunohistochemistry to identify non-specific and specific binding of test molecules, such as antibodies or antibody-like proteins in different types of human or animal tissues, respectively.

Frequently, the staining profiles of animal and human tissues may vary in terms of intensity and distribution. Thereby non-specific and specific binding of test molecules is assessed in order to predict treatment-related toxicity, i.e. toxicity based on non-specific binding. Tissue cross reactivity studies support human clinical trials as a fundamental component of the preclinical safety assessment data package.

Although conventional immunohistochemistry is widely used for tissue cross reactivity studies, this method has some constrains, including high inter-observer variability and the use of only one marker per tissue section (Tan et al, 2020).

There is a need in the art for an improved or alternative method for identifying antibodies or antigen binding fragments thereof that are well-suited for *in vivo* applications.

### Brief description of the invention

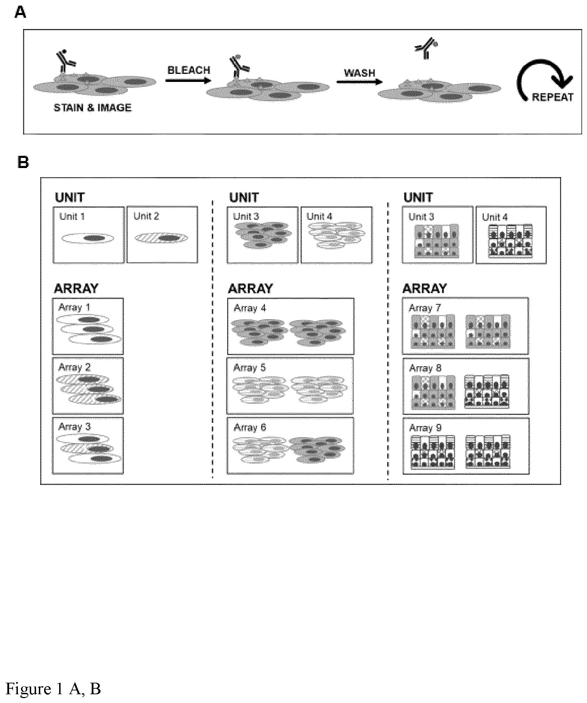

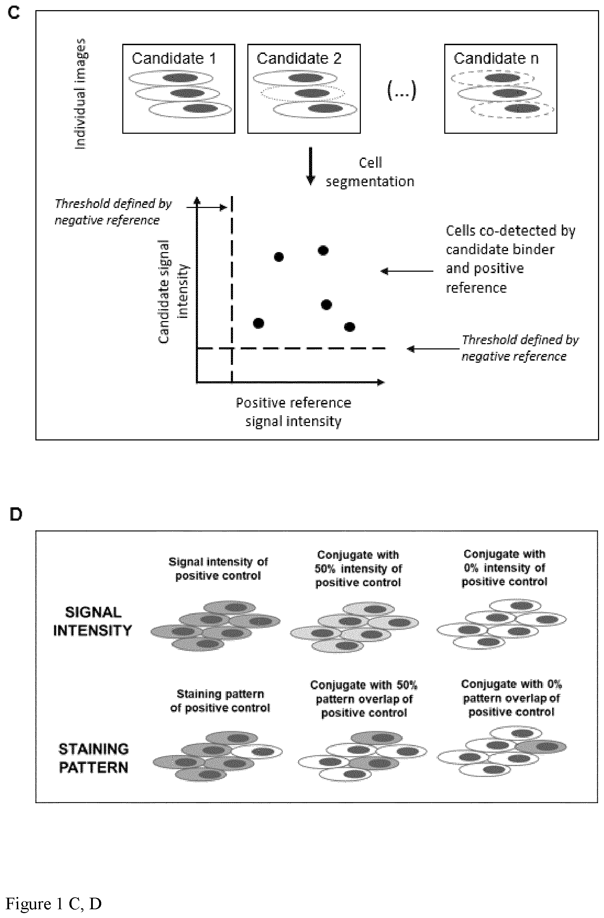

Multiplexing approaches can overcome the limitation of testing a single marker per tissue section. This improvement is of particular interest for the analysis of novel antibodies and antibody-fragments. Additionally, multiplexing enables data generation from sample with limited size or scares availability (Figure 1 A). Image segmentation allows to identify individual cells, i.e. units, from the sample, i.e. the array (Figure 1 B). Subsequent analysis of single cells for co-expression of candidate binder and positive reference allows the identification of target-specific signal of the binder candidate. Here, the candidates have a similar staining pattern as the reference antibodies in terms of distribution (Figure 1 C) indicating antigen-specificity. Multiplexing on the same tissue sample allows to analyze and compare the staining profiles, i.e. signal distribution, of novel antibody-fragments in relation to reference antibodies targeting the same antigen (Figure 1 D).

The *in-vitro* method as disclosed herein allows to assess specificity as well as tissue cross reactivity of novel binders, i.e. antigen recognizing moieties such as antibodies or antigen binding fragments thereof.

The core of the method is that novel binders specific for an antigen are compared with a positive reference that is specific for said antigen present on/in a target structure such as a cancer cell/tumor tissue, wherein the positive reference is a well-known antigen binding domain specific for said antigen, and wherein all binders have been contacted subsequentially with units such as tissues on an aggregate such as a tissue microarray, and wherein every binder comprises a conjugate comprising a fluorescent moiety and an antigen recognizing moiety (an antigen binding moiety) that can bind to said antigen.

For allowing said comparison between said novel binders and said positive reference the method comprises the step of determining
i) the (overall) intensity of the fluorescence signal emitted by the fluorescent moieties of the respective conjugates present in/on each unit of said aggregate, and
ii) the spatial distribution of the intensities of the fluorescence signals emitted by the fluorescent moieties of the respective conjugate present in/on the units of said aggregate, thereby identifying a pattern reflecting the presence of said respective conjugate in/on units within said aggregate.

The comparison is now for the spatial distribution of the intensities of the fluorescence signals between the positive reference and for each of the novel binders. This allows the identification and selection from all conjugates, that are not the positive reference, the conjugates with antigen binding moieties that
have an at least 45%, at least 50%, at least 55%, at least 60%, at least 65%, at least 70%, at least 75%, at least 80%, at least 85%, at least 90%, at least 95% or at least 100% overlap of the pattern relative to the pattern of the positive reference.

The aggregate may comprise units that present the antigen and may have units that do not present said antigen. Figure 1 B shows examples for aggregates.

The process may include also a negative control, i.e. a unit that does not present said antigen.

In one embodiment of the invention the method may be for generating an immune cell comprising a chimeric antigen receptor (CAR) comprising an antigen binding moiety specific for a target antigen expressed on the surface of a target cell by selecting one of the antigen binding moieties identified by the method as disclosed herein as the antigen binding moiety of said CAR. If said target cell is a tumor cell, then the method allows the facilitated selection of suiting binder candidates for use in CAR immune cell therapy with maximal target reactivity and minimal off-tumor and -target reactivity, and safety of the immune cell CAR therapy may be improved.

The fluorescent signals measured or determined within the method as disclosed herein may be performed with a fluorescence detector that measures the intensity of fluorescence radiation emitted by the fluorescent moieties of the conjugates. Such fluorescence detectors are well-known in the art and may be e.g. a fluorescence microscope or e.g. the MACSima^{™} Imaging System (Miltenyi Biotec).

In order to investigate a plurality of potential binders or conjugates, it is preferred to quench the fluorescence radiation emitted by the fluorescent moieties by e.g. enzymatically degrading (disrupting) of the conjugates. Such conjugates are for example disclosed in EP3037821A1, EP16203689.1 or EP16203607.3. In alternative, the fluorescence radiation emitted by the fluorescent moieties may be quenched by radiation.

### Brief description of the drawings

Figure 1: Schematic overviews of the staining and selection principle of candidate binders in comparison to a positive reference. (A) Overview of the staining and imaging procedure of conjugates on cells. (B) Overview of unit and array examples to characterize candidate binders. (C) Overview of the quantification strategy of conjugate staining in comparison to a positive reference clone. (D) Overview of signal intensity and signal patterns.

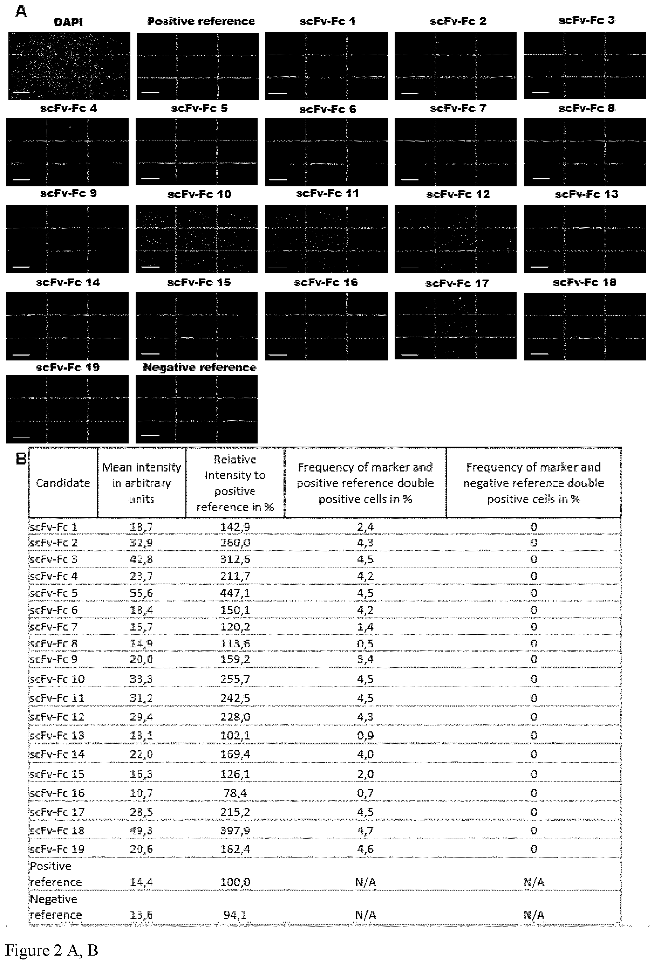

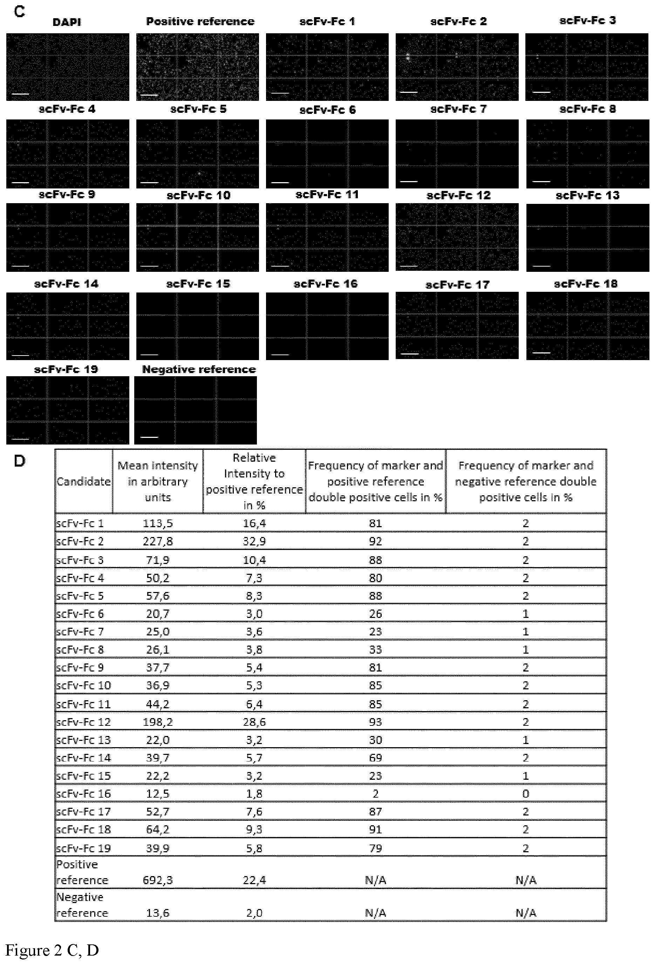

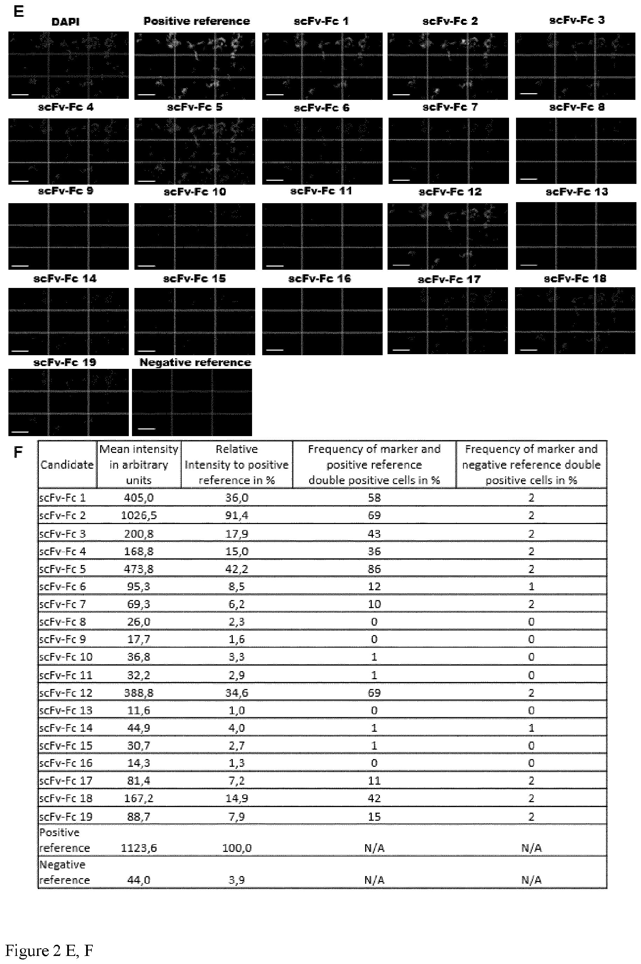

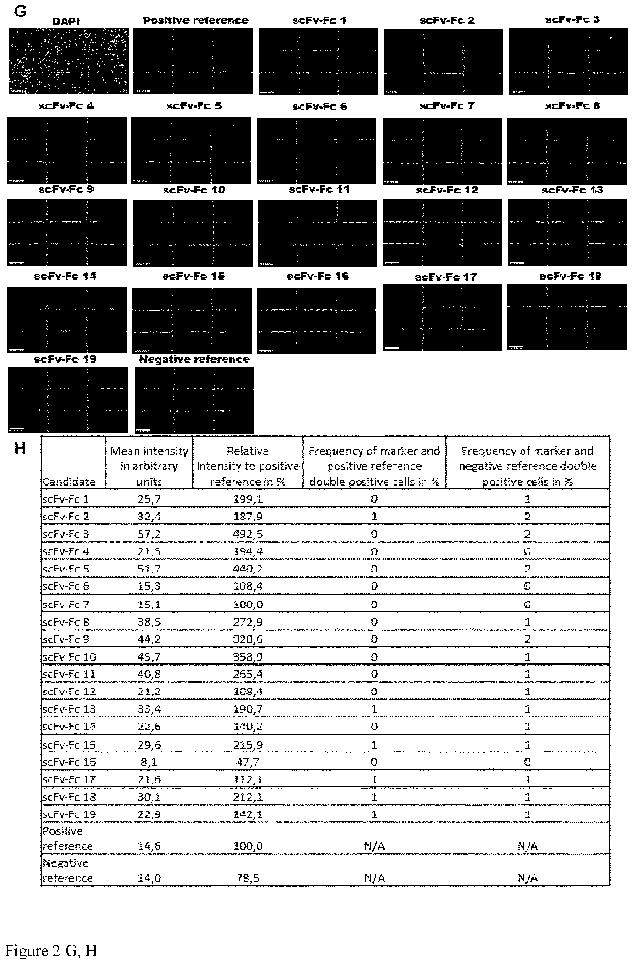

Figure 2: Cyclic imaging of target directed scFv-Fc-PE molecules on human cell lines. Cells were seeded at a concentration of 1E6 (Jurkat) or 2E5 (OV90) cells per 24-well and fixed with 4% PFA before pre-staining with DAPI. Staining of each conjugate was automatically performed, the signal was erased and the sample was washed. A monoclonal antibody directed against the target of interest was used as a positive reference. Cyclic imaging of scFv-Fc-PE conjugates on the cells was performed in a concentration of 5 µg/mL. The scale bar indicates 200 µm. An 20x objective was used and the exposure time was set to 80ms. (A) Staining of target directed scFv-Fc-PE conjugates and a positive reference on target cells that do not express the antigen of interest (Jurkat wt) and (B) quantification of the corresponding signal intensities and signal overlap with the positive reference (C) Staining of target directed scFv-Fc-PE conjugates and a positive reference on cells with a transgenic knock-in of the antigen of interest (Jurkat KI) and (D) quantification of the corresponding signal intensities and signal overlap with the positive reference. (E) Staining of target directed scFv-Fc-PE conjugates and a positive reference on cells with physiological target expression (Ov-90 wt) and (F) quantification of the corresponding signal intensities and signal overlap with the positive reference (G) Staining of target directed scFv-Fc-PE conjugates and a positive reference on cells with a CRISPR/Cas9-mediated knock-out of the antigen of interest (Ov-90 KO) and (H) quantification of the corresponding signal intensities and signal overlap with the positive reference.

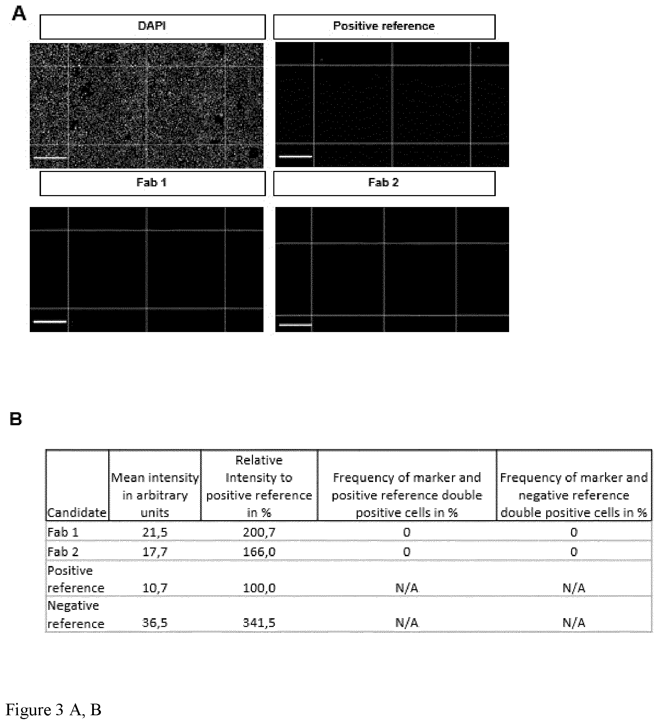

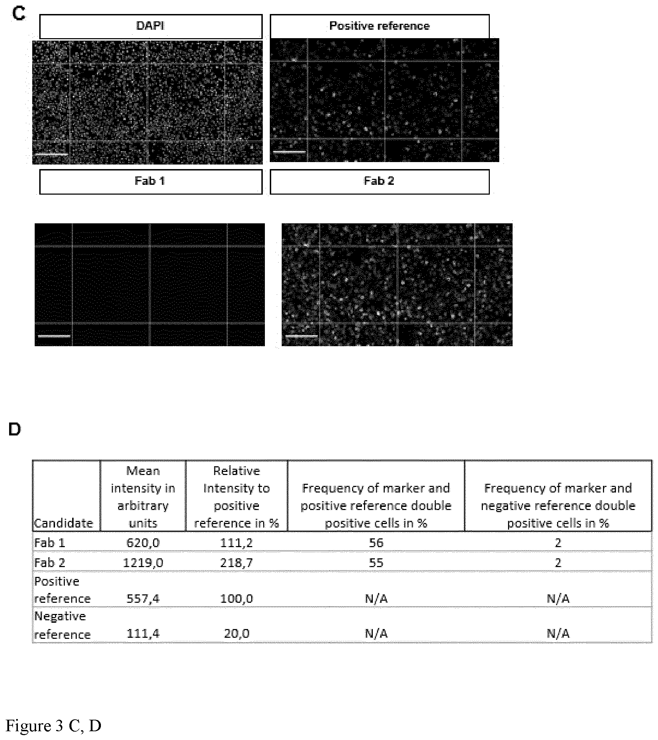

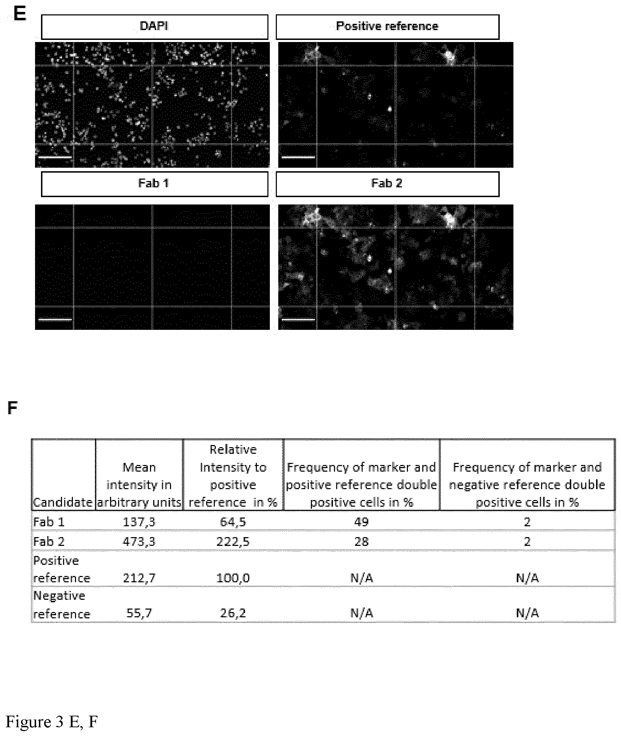

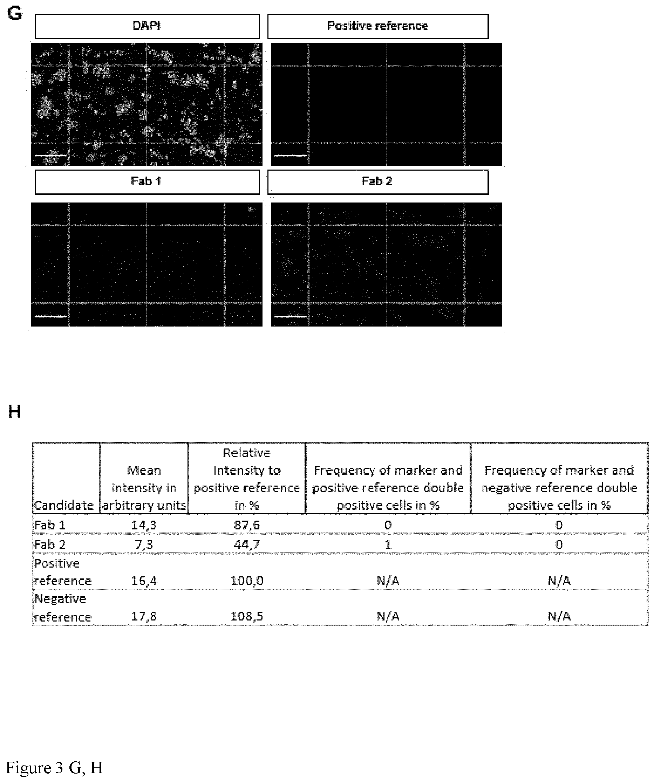

Figure 3: Cyclic imaging of target directed Fab-PE molecules on human cell lines. Cells were seeded at a concentration of 1E6 (Jurkat) or 2E5 (OV90) cells per 24-well and fixed with 4% PFA before pre-staining with DAPI. Staining of each conjugate was automatically performed, the signal was erased and the sample was washed. A monoclonal antibody directed to the target of interest was used as a positive reference. Cyclic imaging of Fab-PE conjugates on cells was performed in a concentration of 5 µg/mL. The scale bar indicates 200 µm. An 20x objective was used and the exposure time was set to 80ms. (A) Staining of target directed Fab-PE conjugates and a positive reference on target cells that do not express the antigen of interest (Jurkat wt). (B) quantification of the corresponding signal intensities and signal overlap with the positive reference. (C) Staining of target directed Fab-PE conjugates and a positive reference on cells with a transgenic knock-in of the antigen of interest (Jurkat KI) and (D) quantification of the corresponding signal intensities and signal overlap with the positive reference. (E) Staining of target directed Fab-PE conjugates and a positive reference on cells with physiological target expression (Ov-90 wt) and (F) quantification of the corresponding signal intensities and signal overlap with the positive reference (G) Staining of target directed Fab-PE conjugates and a positive reference on cells with a CRISPR/Cas9-mediated knock-out of the antigen of interest (Ov90 KO) and (H) quantification of the corresponding signal intensities and signal overlap with the positive reference.

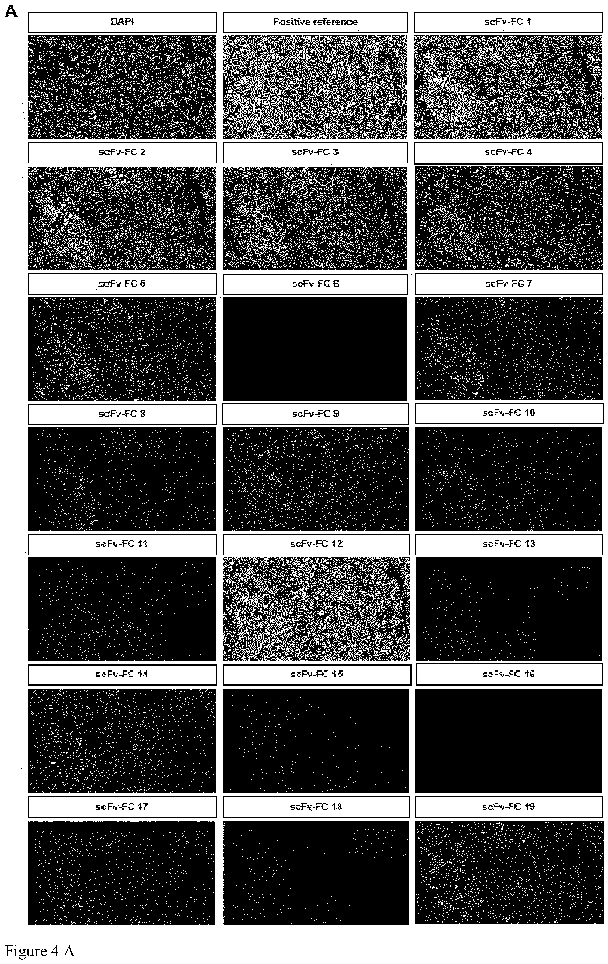

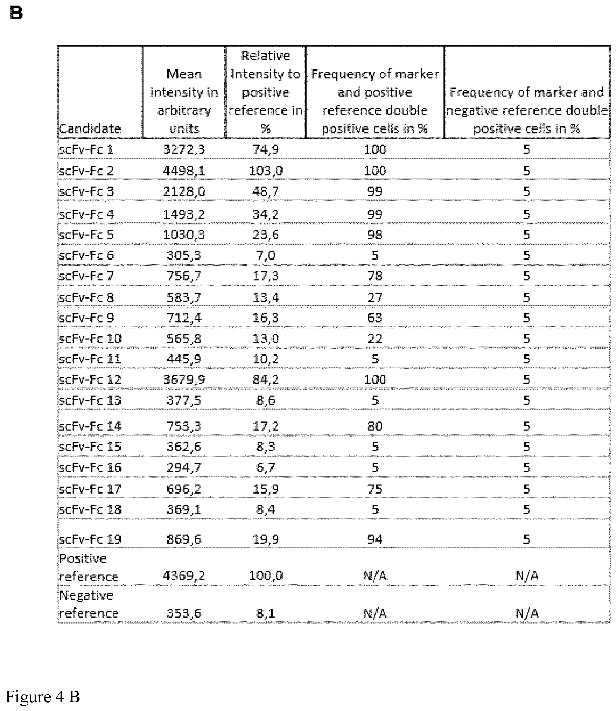

Figure 4: Cyclic imaging of target directed scFv-Fc-PE molecules on human tissue. Cryopreserved tissues were cut into 8 µm sections and fixed with 4% PFA before pre-staining with DAPI. Staining of each conjugate was automatically performed, the signal was erased and the sample was washed. A monoclonal antibody directed to the target of interest was used as a positive reference. Cyclic imaging of scFv-Fc-PE conjugates on the tissue was performed in a concentration of 5 µg/mL. The scale bar indicates 200 µm. An 20x objective was used and the exposure time was set to 640 ms. (A) Staining of target directed scFv-Fc-PE conjugates and a positive reference on target expressing tissue (High-grade serous ovarian cancer) and (B) quantification of the corresponding signal intensities and signal overlap with the positive reference.

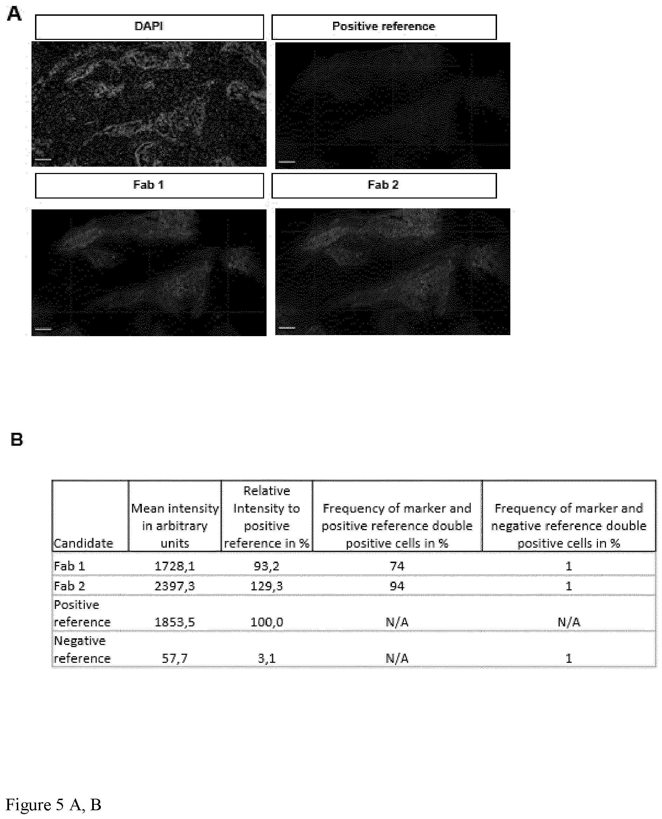

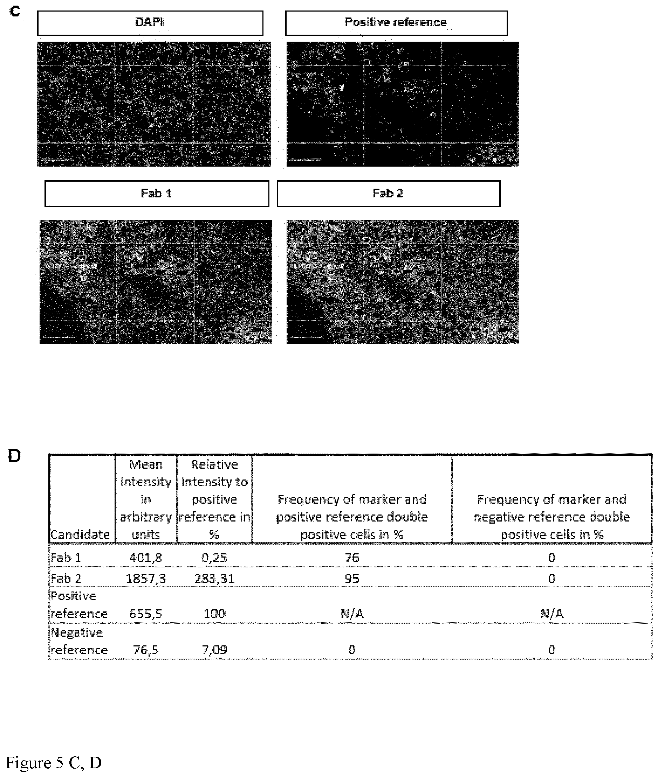

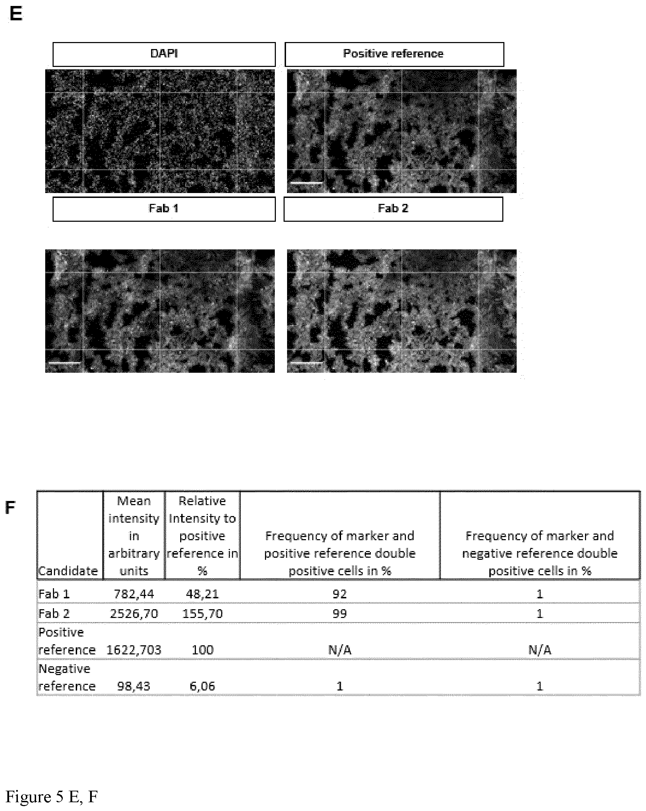

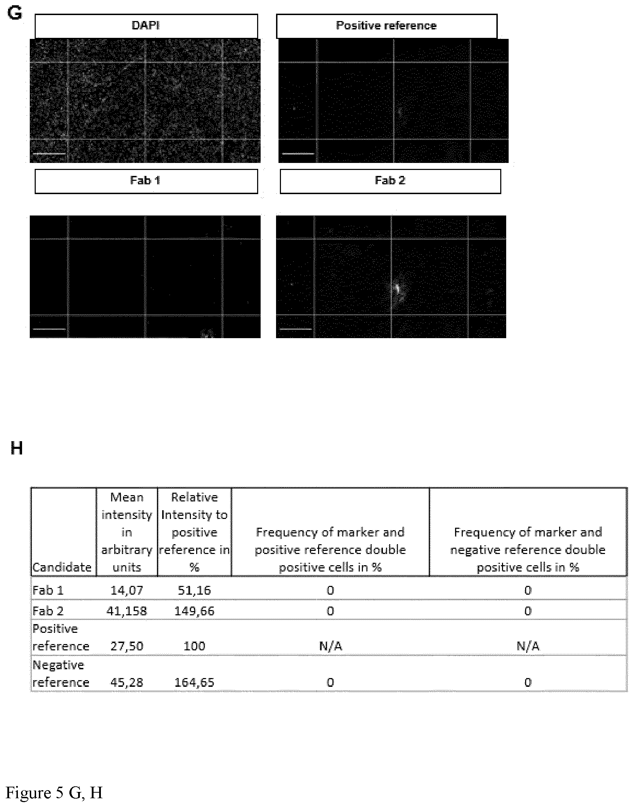

Figure 5: Cyclic imaging of target directed Fab-PE molecules on human tissue. Cryopreserved tissues were cut into 8 µm sections and fixed with 4% PFA before pre-staining with DAPI. Staining of each conjugate was automatically performed, the signal was erased and the sample was washed. A monoclonal antibody directed to the target of interest was used as a positive reference. Cyclic imaging of Fab-PE conjugates on the tissue was performed in a concentration of 5 µg/mL. The scale bar indicates 200 µm. An 20x objective was used and the exposure time was set to 640 ms. (A) Staining of target directed Fab-PE conjugates and a positive reference on target expressing diseased tissue (High-grade serous ovarian cancer) and (B) quantification of the corresponding signal intensities and signal overlap with the positive reference. (C) Staining of target directed Fab-PE conjugates and a positive reference on target expressing healthy tissue (kidney) and (D) quantification of the corresponding signal intensities and signal overlap with the positive reference. (E) Staining of target directed Fab-PE conjugates and a positive reference on target expressing healthy tissue (lung) and (F) quantification of the corresponding signal intensities and signal overlap with the positive reference. (G) Staining of target directed Fab-PE conjugates and a positive reference on healthy tissue with no expression of the target of interest (ovary) and (H) quantification of the corresponding signal intensities and signal overlap with the positive reference).

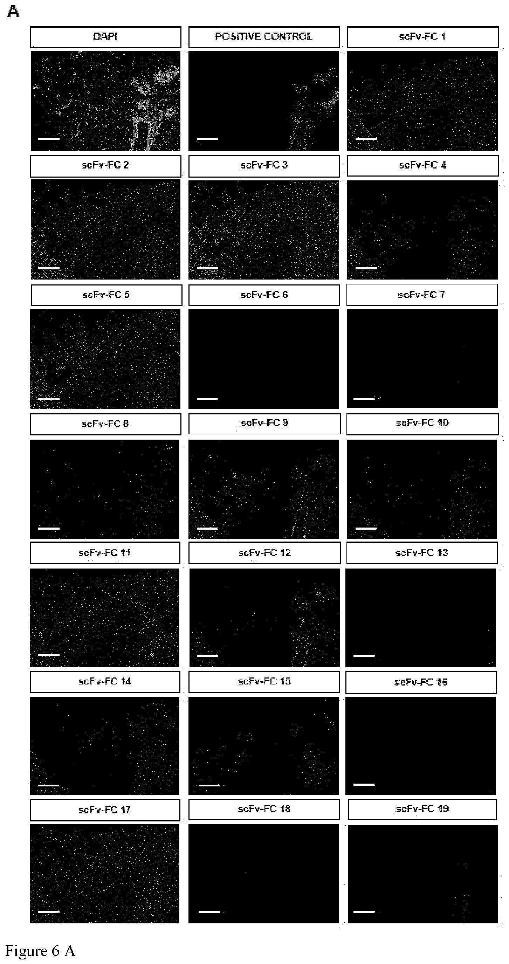

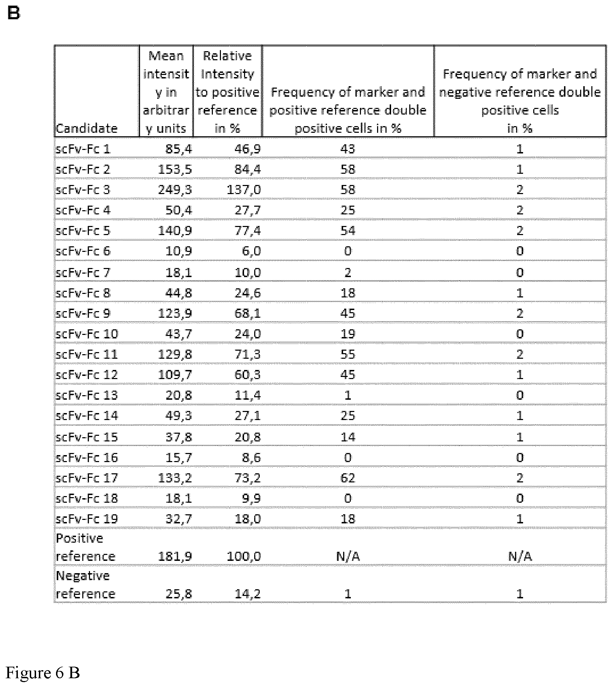

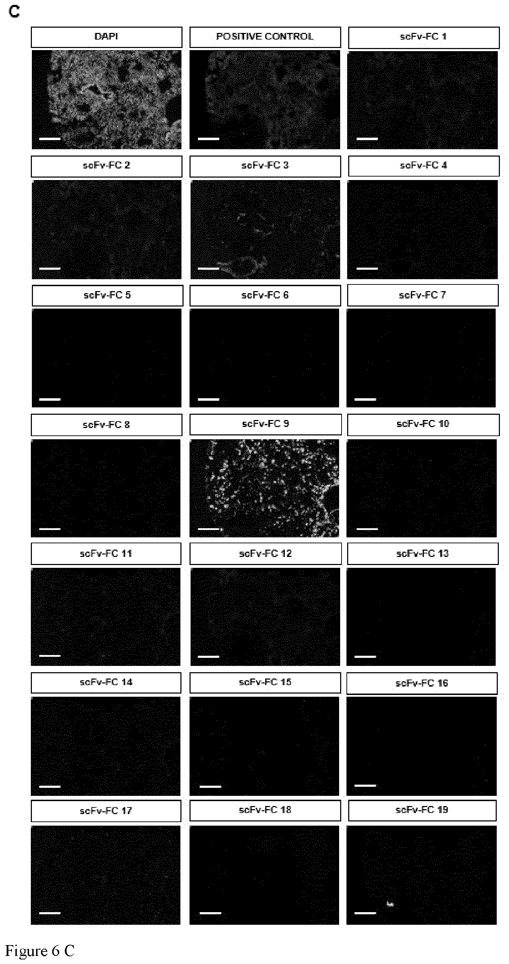

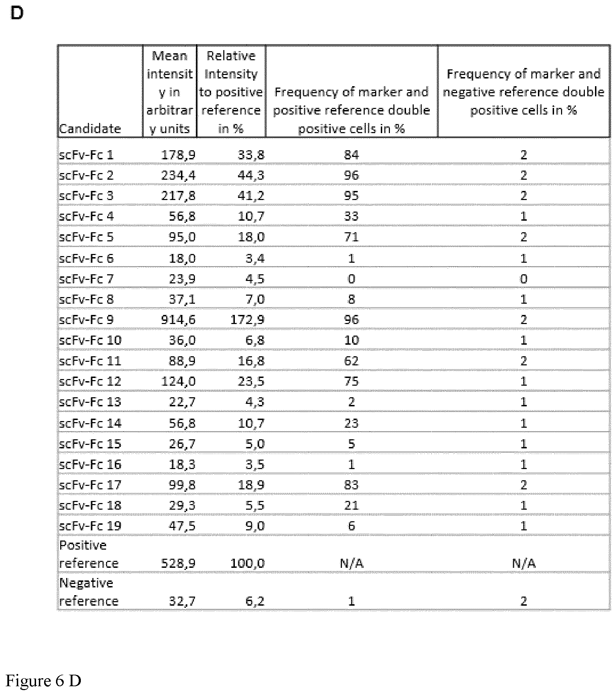

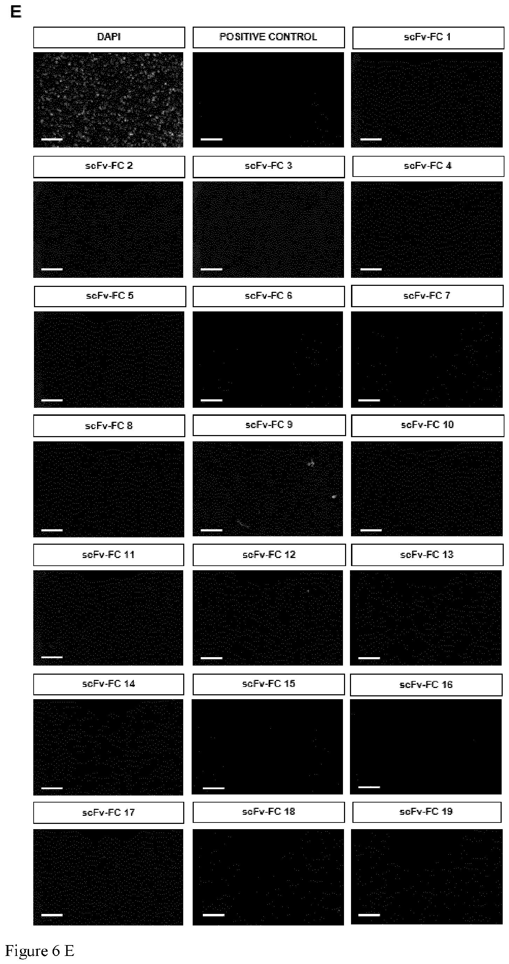

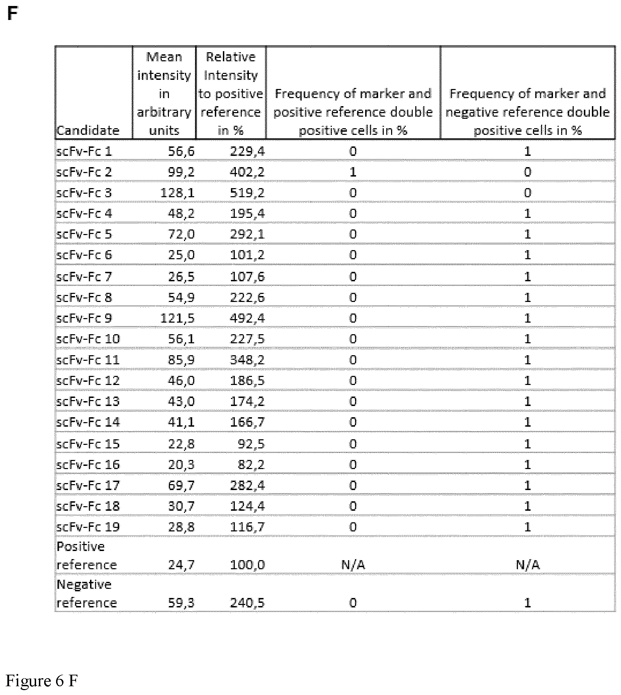

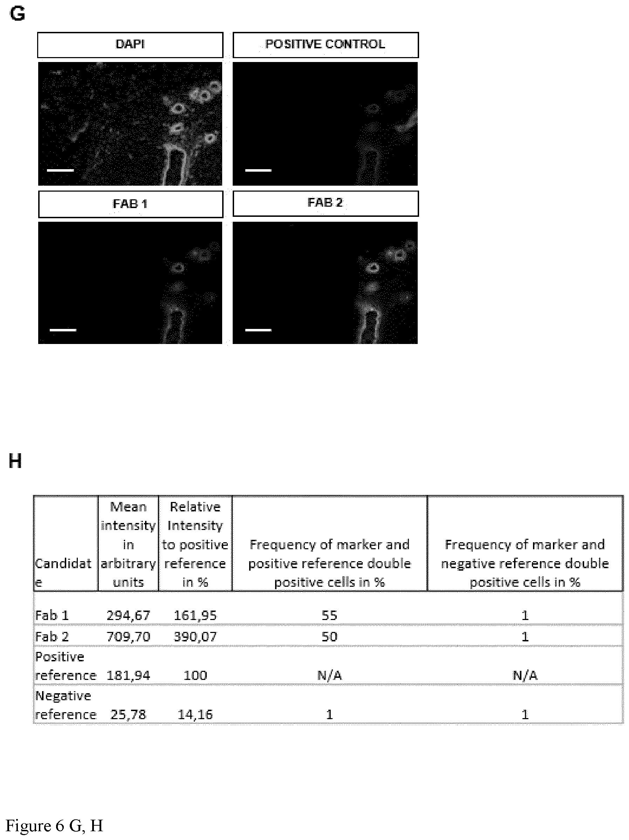

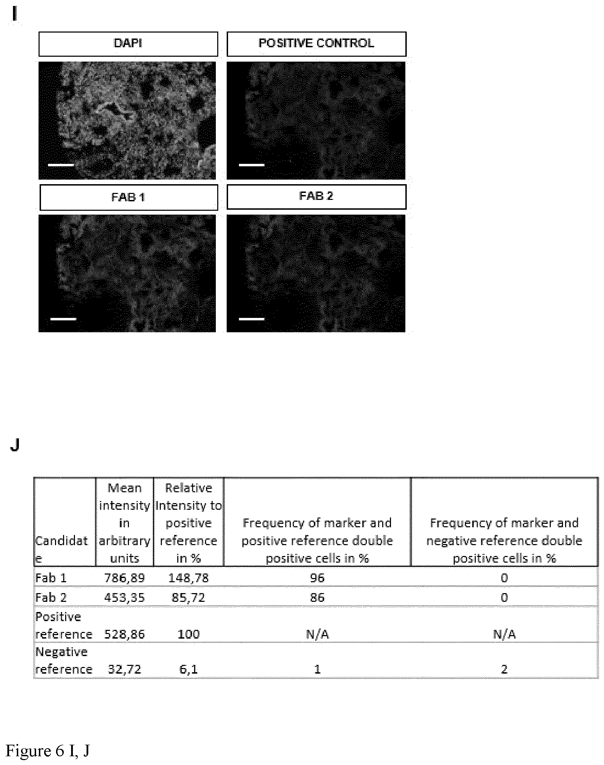

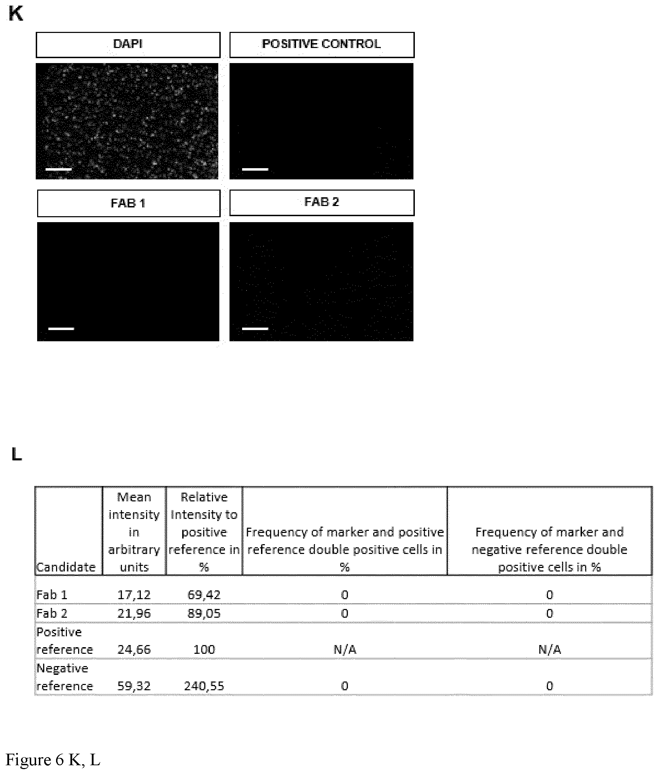

Figure 6: Cyclic imaging of target directed scFv-Fc-PE and Fab-PE molecules on human tissue micro sections. Cryopreserved tissues were fixed with ice cold acetone for 5 minutes before pre-staining with DAPI. Staining of each conjugate was automatically performed, the signal was erased and the sample was washed. A monoclonal antibody directed to the target of interest was used as a positive reference. Cyclic imaging of scFv-Fc and Fab-PE conjugates on the tissue sections was performed in a concentration of 5 µg/mL. The scale bar indicates 200 µm. An 20x objective was used and the exposure time was set to 640 ms. Staining of target directed scFv-Fc-PE conjugates and a positive reference on target expressing healthy tissue as (A) breast and (B) quantification, (C) lung and (D) quantification of the corresponding signal intensities and signal overlap with the positive reference.

Staining of target directed scFv-Fc-PE conjugates and a positive reference on healthy tissue with no expression of the target of interest as (E) brain and (F) quantification of the corresponding signal intensities and signal overlap with the positive reference.

Staining of target directed Fab-PE conjugates and a positive reference on target expressing healthy tissue as (G) breast and (H) quantification and (I) lung and (J) quantification of the corresponding signal intensities and signal overlap with the positive reference.

Staining of target directed Fab-PE conjugates and a positive reference on healthy tissue with no expression of the target of interest as (K) brain and (L) quantification of the corresponding signal intensities and signal overlap with the positive reference.

### Detailed description of the invention

In an aspect the present invention provides an *in-vitro* method for selecting antigen recognizing moieties having specificity for a target structure, the method comprising
a) providing an aggregate comprising spatially separated units comprising
   i) first units that present said target structure, or
   ii) first units that present said target structure and second units that do not present said target structure
b) contacting said aggregate with a first conjugate comprising a fluorescent moiety and a first antigen recognizing moiety that can bind to said target structure,
c) removing unbound first conjugate from said aggregate of step b) and determining
   i) the (overall) intensity of the fluorescence signal emitted by the fluorescent moieties of the first conjugate present in/on each unit of said aggregate, and
   ii) the spatial distribution of the intensities of the fluorescence signals emitted by the fluorescent moieties of the first conjugate present in/on the units of said aggregate, thereby identifying a pattern reflecting the presence of said first conjugate in/on units of said aggregate,
d) repeating steps b) and c) at least once by erasing the preceding fluorescence signal emitted by the fluorescent moieties of the preceding conjugate with at least one further conjugate comprising a fluorescent moiety and a further antigen recognizing moiety that can bind to said target structure, wherein said first conjugate or one of said at least one further conjugates is a positive reference
e) identifying and selecting from the conjugates of step b) to d), that are not the positive reference, the conjugates with antigen recognizing moieties that have an at least 45%, at least 50%, at least 55%, at least 60%, at least 65%, at least 70%, at least 75%, at least 80%, at least 85%, at least 90%, at least 95% or at least 100% overlap of the pattern as determined in step c) ii) relative to the pattern of the positive reference.

Said method, wherein said target structure is a target antigen expressed on the surface of a target cell, and wherein said first units of said aggregate comprise individual units comprising
a) target cells expressing said target antigen on the surface of said target cells, or
b) cells expressing recombinantly said target antigen on the surface of said cells, wherein said cells expressing recombinantly said target antigen are a positive control;
   and optionally in addition to a) or b):
c) non-target cells or tissue that express said target antigen on the surface of said non-target cells or of the cells of said tissue;
and wherein said second units of said aggregate comprise individual units comprising non-target cells or tissue that do not present said target antigen on the surface of said non target cells or of the cells of said tissue, wherein each individual unit comprise only one type of non-target cells or one type of tissue.

Said target cells may be cancer cells (or tumor cells) or tumor tissue.

Said target antigen may be a tumor associated antigen (TAA).

Said tissue may be healthy tissue, preferentially human healthy tissue or cancer cells (or tumor cells) of another type of cancer than the cancer cells that are the target cells.

Said repeating steps b) and c) with further conjugates comprising a fluorescent moiety and further antigen recognizing moieties that can bind to said target structure may be at least 2-fold, at least 5-fold, at least 10-fold, at least 15-fold, at least 20-fold, at least 30-fold, at least 40-fold, at least 50-fold, at least 60-fold, at least 70-fold, at least 80-fold, at least 90-fold, or at least 100-fold.

Said at least one further conjugate comprising a fluorescent moiety and a further antigen recognizing moiety that can bind to said target structure may be at least 2, at least 5, at least 10, at least 15, at least 20, at least 30, at least 40, at least 50, at least 60, at least 70, at least 80, at least 90, or at least 100 further conjugates, and wherein said further antigen recognizing moieties of said further conjugates may be different from each other.

The fluorescent moieties of said first conjugate comprising a fluorescent moiety and a first antigen recognizing moiety and of said at least one further conjugate comprising a fluorescent moiety and a further antigen recognizing moiety may be the same fluorescent moieties or may be different moieties.

If in step b) and/or step d) said aggregate is contacted simultaneously with more than one conjugate comprising a fluorescent moiety and an antigen recognizing moiety, then each of said fluorescent moieties of said conjugates that are contacted simultaneously with said aggregate has a different fluorescent moiety.

The terms "antigen recognizing moiety" or antigen binding moiety may be used interchangeably. Said antigen binding moiety may be an antibody or antigen binding fragment thereof. Said antigen binding fragment thereof may be a Fab, scFv or nanobody.

Said aggregate may be a cluster (or a collection of) of first and second units on one solid phase such as a microarray (tissue microarray) or such as a specimen slide or said aggregate may be a collection of first and second units that are all or partly on different solid phases such as on different specimen slides allowing also a timely separated processing of the units of the aggregate that are not on the same solid phase.

A unit of the aggregate may be a defined spot on the aggregate, wherein said spot may comprise one type of cells or one type of tissue.

Said method, wherein said second units of said aggregate comprise additionally an individual unit comprising cells that are recombinantly knocked-out for said target antigen as a negative control.

Said method, wherein said second units of said aggregate comprise at least 2, at least 3, at least 4, at least 5, at least 6, at least 7, at least 8, at least 9, or at least 10 individual units having different types of non-target cells or different types of tissues, respectively.

Said method, wherein said *in-vitro* method comprises additionally the steps:
A) contacting said aggregate with a conjugate comprising a fluorescent moiety and an antigen recognizing moiety that does not bind to said target structure as a negative reference, and
B) removing unbound conjugate from said aggregate of step A) and determining
   i) the intensity of the fluorescence signal emitted by the fluorescent moieties of said conjugate, that is the negative reference, present in/on each unit of said aggregate, and
   ii) the spatial distribution of the intensities of the fluorescence signals emitted by the fluorescent moieties of said conjugate, that is the negative reference, present in/on the units of said aggregate, thereby identifying a pattern reflecting the presence of said conjugate, that is the negative reference, in/on units within said aggregate;
   and identifying and selecting in step e) additionally from the conjugates of step b) to d), that are not the positive reference, the conjugates with antigen recognizing moieties that
   i) have a mean intensity more than the mean intensity of the fluorescence signal as determined in step c) i) relative to the intensity of the negative reference, and
   ii) have a pattern that does not overlap more than 5% with the pattern as determined in step c) ii) relative to the pattern of the negative reference.

Said method, wherein said an *in-vitro* method additionally comprises the step (after step e)) of providing (supplying) an immune cell with a chimeric antigen receptor (CAR) comprising
a) an antigen recognizing moiety
b) a transmembrane domain
c) an intracellular signaling domain,
wherein the antigen recognizing moiety of the CAR is an antigen recognizing moiety identified and selected in step e).

Said CAR, wherein said intracellular signaling domain comprises a stimulatory domain and/or a co-stimulatory domain. Said stimulatory domain may be e.g. the stimulatory domain of CD3zeta. Said co-stimulatory domain may be e.g. the co-stimulatory domain of CD28 or 4-1BB.

The provision of said immune cell with said chimeric antigen receptor may be achieved by transduction of the immune cell with a viral vector, e.g. a retroviral vector such as a lentiviral vector comprising a nucleic acid encoding said CAR.

Said method, wherein erasing of the preceding fluorescence signal is achieved by enzymatical disruption of a biodegradable linker used between the fluorescent moiety and the antigen recognizing moiety of the conjugate.

Said method, wherein said removing unbound conjugate from said aggregate is achieved by a washing step.

Said method, wherein erasing of the preceding fluorescence signal is achieved by bleaching of the fluorescent moiety. Said bleaching may be performed by photobleaching, enzymatical disruption and/or chemical bleaching.

In one embodiment of the invention the method comprises a method for generating an immune cell comprising a chimeric antigen receptor (CAR) comprising an antigen recognizing moiety specific for a target antigen expressed on the surface of a target cell, the method comprising:
a) providing an aggregate comprising spatially separated units comprising
   i) first units, wherein said first units of said aggregate comprise individual units comprising
      A) target cells expressing said target antigen on the surface of said target cells, or
      B) cells expressing recombinantly said target antigen on the surface of said cells, wherein said cells expressing recombinantly said target antigen are a positive control;
         and optionally in addition to A) or B):
      C) non-target cells or tissue that express said target antigen on the surface of said non-target cells or of the cells of said tissue,
   ii) second units, wherein said second units of said aggregate comprise individual units comprising non-target cells or tissue that do not present said target antigen on the surface of said non target cells or of the cells of said tissue, wherein each individual unit comprise only one type of non-target cells or one type of tissue, and
b) contacting said aggregate with a first conjugate comprising a fluorescent moiety and a first antigen recognizing moiety that can bind to said target structure,
c) removing unbound first conjugate from said aggregate of step b) and determining
   i) the intensity of the fluorescence signal emitted by the fluorescent moieties of the first conjugate present in/on each unit of said aggregate, and
   ii) the spatial distribution of the intensities of the fluorescence signals emitted by the fluorescent moieties of the first conjugate present in/on the units of said aggregate, thereby identifying a pattern reflecting the presence of said first conjugate in/on units of said aggregate,
d) repeating steps b) and c) at least once by erasing the preceding fluorescence signal emitted by the fluorescent moieties of the preceding conjugate with at least one further conjugate comprising a fluorescent moiety and a further antigen recognizing moiety that can bind to said target structure, wherein said first conjugate or one of said at least one further conjugates is a positive reference
e) identifying and selecting from the conjugates of step b) to d), that are not the positive reference, the conjugates with antigen recognizing moieties that have an at least 45%, at least 50%, at least 55%, at least 60%, at least 65%, at least 70%, at least 75%, at least 80%, at least 85%, at least 90%, at least 95% or at least 100% overlap of the pattern as determined in step c) ii) relative to the pattern of the positive reference,
f) providing (supplying) an immune cell with a chimeric antigen receptor (CAR) comprising
   I) an antigen recognizing moiety
   II) a transmembrane domain
   III) an intracellular signaling domain,
wherein the antigen recognizing moiety of the CAR is an antigen recognizing moiety identified and selected in step e).

Said immune cell may be e.g. a T cell, an NK cell or a gamma/delta T cell.

In another aspect the present invention provides an immune cell expressing a CAR comprising
I) an antigen recognizing moiety
II) a transmembrane domain, and
III) an intracellular signaling domain,
wherein the antigen recognizing moiety of the CAR is obtained by the methods as disclosed herein.

In another aspect the present invention provides an immune cell expressing a CAR for use in treatment of a disease such as cancer, an autoimmune disease or an infectious disease in a subject in need thereof, said CAR comprising
I) an antigen recognizing moiety
II) a transmembrane domain, and
III) an intracellular signaling domain,
wherein the antigen recognizing moiety of the CAR is obtained by the methods as disclosed herein.

In another aspect the present invention provides a pharmaceutical composition comprising
A) an immune cell expressing a CAR, said CAR comprising
   I) an antigen recognizing moiety
   II) a transmembrane domain, and
   III) an intracellular signaling domain,
   wherein the antigen recognizing moiety of the CAR is obtained by the methods as disclosed herein, and optionally
B) a pharmaceutically acceptable carrier.

Pharmaceutically acceptable carriers, diluents or excipients may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives.

All definitions, characteristics and embodiments defined herein with regard to the first aspect of the invention as disclosed herein also apply mutatis mutandis in the context of the other aspects of the invention as disclosed herein.

### Definitions

Unless defined otherwise, technical and scientific terms used herein have the same meaning as commonly understood by one of ordinary skill in the art to which this invention belongs. As used herein the term "comprising" or "comprises" is used in reference to compositions, methods, and respective component(s) thereof, that are essential to the method or composition, yet open to the inclusion of unspecified elements, whether essential or not.

The term "enzymatical disruption" as used herein in the context of disruption of the conjugates as disclosed herein refers to antibodies or antigen binding fragments thereof specific for the antigen as disclosed herein that are directly or indirectly linked via a biodegradable spacer and wherein said biodegradable spacer may be specifically biodegraded, digested or cut by the activity of an enzyme and thereby split the fluorescent moiety and the antigen recognizing moiety of the conjugate in at least two separate molecules. In addition, if said antibody or antigen binding fragment thereof such as said Fab has low affinity and/or a high k(off) rate said antibody or antigen binding fragment thereof such as said Fab will be removed from the antigen to that is has bound.

The enzymatically degradable spacer P can be any molecule which can be cleaved by a specific enzyme, especially a hydrolase. Suitable as enzymatically degradable spacer P are, for example, polysaccharides, proteins, peptides, depsipeptides, polyesters, nucleic acids, and derivatives thereof as disclosed e.g. in EP3037821A1.

Suitable polysaccharides are, for example, dextrans, pullulans, inulins, amylose, cellulose, hemicelluloses, such as xylan or glucomannan, pectin, chitosan, or chitin, which may be derivatized to provide functional groups for covalent or non-covalent binding of the fluorescent moiety X and the antigen recognizing moiety Y. A variety of such modifications are known in the art, for example, imidazolyl carbamate groups may be introduced by reacting the polysaccharide with N,N'-carbonyl diimidazole. Subsequently amino groups may be introduced by reacting said imidazolyl carbamate groups with hexane diamine. Polysaccharides may also be oxidized using periodate to provide aldehyde groups or with N,N'-dicyclohexylcarbodiimide and dimethylsulfoxide to provide ketone groups. Aldehyde or ketone functional groups can be reacted subsequently preferably under conditions of reductive amination either with diamines to provide amino groups or directly with amino substituents on a proteinaceous binding moiety. Carboxymethyl groups may be introduced by treating the polysaccharide with chloroacetic acid. Activating the carboxy groups with methods known in the art which yield activated esters such N-hydroxysuccinimid ester or tetrafluorophenyl ester allows for reaction with amino groups either of a diamine to provide amino groups or directly with an amino group of a proteinaceous binding moiety. It is generally possible to introduce functional group bearing alkyl groups by treating polysaccharides with halogen compounds under alkaline conditions. For example, allyl groups can be introduced by using allyl bromide. Allyl groups can further be used in a thio|-ene reaction with thiol bearing compounds such as cysteamine to introduce amino groups or directly with a proteinaceous binding moiety with thiol groups liberated by reduction of disulfide bonds or introduced by thiolation for instance with 2-iminothiolane.

Proteins, peptides, and depsipeptides used as enzymatically degradable spacer P can be functionalized via side chain functional groups of amino acids to attach fluorescent moiety X and antigen recognizing moiety Y. Side chains functional groups suitable for modification are for instance amino groups provided by lysine or thiol groups provided by cysteine after reduction of disulfide bridges.

Polyesters and polyesteramides used as enzymatically degradable spacer P can either be synthesized with co-monomers, which provide side chain functionality or be subsequently functionalized. In the case of branched polyesters functionalization can be via the carboxyl or hydroxyl end groups. Post polymerization functionalization of the polymer chain can be, for example, via addition to unsaturated bonds, i.e. thiolene reactions or azide-alkine reactions, or via introduction of functional groups by radical reactions.

Nucleic acids used as enzymatically degradable spacer P are preferably synthesized with functional groups at the 3' and 5' termini suitable for attachment of the fluorescent moiety X and antigen recognizing moiety Y. Suitable phosphoramidite building blocks for nucleic acid synthesis providing for instance amino or thiol functionalities are known in the art.

The enzymatically degradable spacer P can be composed of more than one different enzymatically degradable units, which are degradable by the same or different enzyme.

Suitable fluorescent moieties are those known from the art of immunofluorescence technologies, e.g., flow cytometry or fluorescence microscopy.

Useful fluorescent moieties might be protein-based, such as phycobiliproteins, polymeric, such as polyfluorenes, small organic molecule dyes, such as Xanthenes, like fluorescein, or rhodamines, cyanines, oxazines, coumarins, acridines, oxadiazoles, pyrenes, pyrromethenes, or metallo-organic complexes, such as Ru, Eu, Pt complexes. Besides single molecule entities, clusters of fluorescent proteins or small organic molecule dyes, as well as nanoparticles, such as quantum dots, upconverting nanoparticles, gold nanoparticles, dyed polymer nanoparticles can also be used as fluorescent moieties.

The process of photobleaching can erase the fluorescent property of the fluorescent moiety as used herein. Photobleaching is the photochemical alteration of a fluorophore molecule such that it is permanently unable to fluoresce. This is caused by cleaving of covalent bonds or non-specific reactions between the fluorophore and surrounding molecules, e.g. by radiation. Target antigens labeled with the conjugate comprising the fluorochrome and the antigen binding moiety may be detected by exciting the fluorescent moiety X and analysing the resulting fluorescence signal. The wavelength of the excitation is usually selected according to the absorption maximum of the fluorescent moiety X and provided by LASER or LED sources as known in the art. If several different fluorescent moieties X are used for multiple colour/parameter detection, care should be taken to select fluorescent moieties having not overlapping absorption spectra, at least not overlapping absorption maxima. Fluorescent moieties bound to the antigen during the method as disclosed herein may be detected, e.g., under a fluorescence microscope, in a flow cytometer, a spectrofluorometer, or a fluorescence scanner. In a preferred embodiment of the invention, the method is used with the MACSima^{™} Imaging System (Miltenyi Biotec).

The term "positive control" as used herein refers to a unit of the aggregate that consciously presents the target structure, e.g. target cells expressing the target antigen on the surface of said target cells or cells expressing recombinantly the target antigen on the surface of said cells. The term "negative control" as used herein refers to a unit of the aggregate that consciously does not present the target structure, e.g. cells that are recombinantly knocked-out for said target antigen.

The term "positive reference" as used herein refers to a conjugate comprising a fluorescent moiety and an antigen recognizing moiety that is known to be able to bind to the target structure. This antigen recognizing moiety of said conjugate, that is a positive reference, may normally be an antibody or antigen binding fragment thereof with known binding characteristics such as known K_{D}, k_{off} and/or k*on* values.

The term "negative reference" as used herein refers to a conjugate comprising a fluorescent moiety and an antigen recognizing moiety that is known to be not able to bind to the target structure. The specificity of this antigen binding moiety that may be an antibody or antigen binding fragment thereof may bind to a known target structure that may be known to be absent in the units of the aggregate.

The terms "specifically binds to" or "specific for" with respect to an antigen-binding domain of an antibody or fragment thereof (e.g. a scFv) refer to an antigen-binding domain which recognizes and binds to a specific antigen, but does not substantially recognize or bind other antigens in a sample. An antigen-binding domain that binds specifically to an antigen from one species may bind also to the homologous antigen from another species. This cross-species reactivity is typical to many antibodies and therefore not contrary to the definition of that antigen-binding domain as specific. An antigen-binding domain that specifically binds to an antigen may bind also to different allelic forms of the antigen (allelic variants, splice variants, isoforms etc.) or homologous variants of this antigen from the same gene family. These cross reactivities are typical to many antibodies and therefore not contrary to the definition of that antigen-binding domain as specific. An antigen-binding domain that specifically binds to an antigen may bind also to a limited number of completely different structures, known as mimotopes. This reactivity is typical to many antibodies and therefore not contrary to the definition of that antigen-binding domain as specific.

The term "antibody" as used herein is used in the broadest sense to cover the various forms of antibody structures including but not being limited to monoclonal and polyclonal antibodies (including full length antibodies), multispecific antibodies (e.g. bispecific antibodies), antibody fragments, i.e. antigen binding fragments of an antibody, immunoadhesins and antibody-immunoadhesin chimeras, that specifically recognize (i.e. bind) a target antigen. "Antibody fragments" comprise a portion of a full length antibody, preferably the variable domain thereof, or at least the antigen binding site thereof ("an antigen binding fragment of an antibody"). Examples of antibody fragments include Fab (fragment antigen binding), scFv (single chain fragment variable), single domain antibodies (VHH, nanobodies), diabodies, dsFv, Fab', diabodies, single-chain antibody molecules, and multispecific antibodies formed from antibody fragments.

The term "transduction" or " transducing" as used herein refers to the genetic modification of cells by retroviral vectors particles encoding (therapeutic) transgenes, such that the retroviral vector binds to the cell, releases the capsid into the cytoplasm, enters the nucleus and the reverse-transcribed retroviral vector genome comprising the transgene is stably integrated into the host cell genome and subsequently expressed by the transduced cell.

Immunotherapy is a medical term defined as the "treatment of disease by inducing, enhancing, or suppressing an immune response". Immunotherapies designed to elicit or amplify an immune response are classified as activation immunotherapies, while immunotherapies that reduce or suppress are classified as suppression immunotherapies. Cancer immunotherapy as an activating immunotherapy attempts to stimulate the immune system to reject and destroy tumors. Adoptive cell transfer uses cell-based, preferentially T cell-based or NK cell-based cytotoxic responses to attack cancer cells. T cells that have a natural or genetically engineered reactivity to a patient's cancer are generated *in-vitro* and then transferred back into the cancer patient. Then the immunotherapy is referred to as "CAR immunotherapy" or in case of use of T cells only as "CAR T cell therapy" or "CAR T cell immunotherapy".

The term "treatment" as used herein means to reduce the frequency or severity of at least one sign or symptom of a disease.

As used herein, the term "subject" refer to an animal. Preferentially, the subject is a mammal such as mouse, rat, cow, pig, goat, chicken dog, monkey or human. More preferentially, the individual is a human. The subject may be a subject suffering from a disease such as cancer (a patient) having malignant cells.

The term "expression" as used herein is defined as the transcription and/or translation of a particular nucleotide sequence driven by its promoter in a cell.

The terms "engineered cell" and "genetically modified cell" as used herein can be used interchangeably. The terms mean containing and/or expressing a foreign gene or nucleic acid sequence which in turn modifies the genotype or phenotype of the cell or its progeny. Especially, the terms refer to the fact that cells, preferentially T cells can be manipulated by recombinant methods well known in the art to express stably or transiently peptides or proteins which are not expressed in these cells in the natural state. For example, T cells, preferentially human T cells are engineered to express an artificial construct such as a chimeric antigen receptor on their cell surface. For example, the CAR sequences may be delivered into cells using a retroviral or lentiviral vector.

The term "tumor" is known medically as a neoplasm. Not all tumors are cancerous; benign tumors do not invade neighboring tissues and do not spread throughout the body.

The term "cancer" is known medically as a malignant neoplasm. Cancer is a broad group of diseases involving unregulated cell growth and includes all kinds of leukemia. In cancer, cells (cancerous cells) divide and grow uncontrollably, forming malignant tumors, and invading nearby parts of the body. The cancer may also spread to more distant parts of the body through the lymphatic system or bloodstream. There are over 200 different known cancers that affect humans.

In general, a CAR may comprise an extracellular domain (extracellular part) comprising the antigen binding domain (antigen recognizing moiety, antigen binding moiety), a transmembrane domain and a cytoplasmic signaling domain (intracellular signaling domain). The extracellular domain may be linked to the transmembrane domain by a linker (or spacer or hinge or hinge region). The extracellular domain may also comprise a signal peptide.

A "signal peptide" refers to a peptide sequence that directs the transport and localization of the protein within a cell, e.g. to a certain cell organelle (such as the endoplasmic reticulum) and/or the cell surface.

Generally, an "antigen binding domain" refers to the region of the CAR that specifically binds to an antigen, e.g. to a tumor associated antigen (TAA) or tumor specific antigen (TSA). The CARs may comprise one or more antigen binding domains (e.g. a tandem CAR). Generally, the targeting regions on the CAR are extracellular. The antigen binding domain may comprise an antibody or an antigen binding fragment thereof. The antigen binding domain may comprise, for example, full length heavy chain, Fab fragments, single chain Fv (scFv) fragments, divalent single chain antibodies, nanobodies (or VHH or single domain antibody) or diabodies. Any molecule that binds specifically to a given antigen such as affibodies or ligand binding domains from naturally occurring receptors may be used as an antigen binding domain. Often the antigen binding domain is a scFv. Normally, in a scFv the variable regions of an immunoglobulin heavy chain and light chain are fused by a flexible linker to form a scFv. Such a linker may be for example the "(G₄/S)₃-linker".

In some instances, it is beneficial for the antigen binding domain to be derived from the same species in which the CAR will be used in. For example, when it is planned to use it therapeutically in humans, it may be beneficial for the antigen binding domain of the CAR to comprise a human or humanized antibody or antigen binding fragment thereof. Human or humanized antibodies or antigen binding fragments thereof can be made by a variety of methods well known in the art.

"Spacer" or "hinge" as used herein refers to the hydrophilic region which is between the antigen binding domain and the transmembrane domain. The CARs may comprise an extracellular spacer domain but is it also possible to leave out such a spacer. The spacer may include e.g. Fc fragments of antibodies or fragments thereof, hinge regions of antibodies or fragments thereof, CH2 or CH3 regions of antibodies, accessory proteins, artificial spacer sequences or combinations thereof. A prominent example of a spacer is the CD8alpha hinge.

The transmembrane domain of the CAR may be derived from any desired natural or synthetic source for such domain. When the source is natural the domain may be derived from any membrane-bound or transmembrane protein. The transmembrane domain may be derived for example from CD8alpha or CD28. When the key signaling and antigen recognition modules (domains) are on two (or even more) polypeptides then the CAR may have two (or more) transmembrane domains. The splitting key signaling and antigen recognition modules enable for a small molecule-dependent, titratable and reversible control over CAR cell expression (e.g. WO2014127261A1) due to small molecule-dependent heterodimerizing domains in each polypeptide of the CAR.

The cytoplasmic signaling domain (the intracellular signaling domain or the activating endodomain) of the CAR is responsible for activation of at least one of the normal effector functions of the immune cell in which the CAR is expressed, if the respective CAR is an activating CAR (normally, a CAR as described herein refers to an activating CAR, otherwise it is indicated explicitly as an inhibitory CAR (iCAR)). "Effector function" means a specialized function of a cell, e.g. in a T cell an effector function may be cytolytic activity or helper activity including the secretion of cytokines. The intracellular signaling domain refers to the part of a protein which transduces the effector function signal and directs the cell expressing the CAR to perform a specialized function. The intracellular signaling domain may include any complete, mutated or truncated part of the intracellular signaling domain of a given protein sufficient to transduce a signal which initiates or blocks immune cell effector functions.

Prominent examples of intracellular signaling domains for use in the CARs include the cytoplasmic signaling sequences of the T cell receptor (TCR) and co-receptors that initiate signal transduction following antigen receptor engagement.

Generally, T cell activation can be mediated by two distinct classes of cytoplasmic signaling sequences, firstly those that initiate antigen-dependent primary activation through the TCR (primary cytoplasmic signaling sequences, primary cytoplasmic signaling domain) and secondly those that act in an antigen-independent manner to provide a secondary or co-stimulatory signal (secondary cytoplasmic signaling sequences, co-stimulatory signaling domain). Therefore, an intracellular signaling domain of a CAR may comprise one or more primary cytoplasmic signaling domains and/or one or more secondary cytoplasmic signaling domains.

Primary cytoplasmic signaling domains that act in a stimulatory manner may contain ITAMs (immunoreceptor tyrosine-based activation motifs).

Examples of ITAM containing primary cytoplasmic signaling domains often used in CARs are that those derived from TCRζ (CD3ζ), FcRgamma, FcRbeta, CD3gamma, CD3delta, CD3epsilon, CD5, CD22, CD79a, CD79b, and CD66d. Most prominent is sequence derived from CD3ζ.

The cytoplasmic domain of the CAR may be designed to comprise the CD3ζ signaling domain by itself or combined with any other desired cytoplasmic domain(s). The cytoplasmic domain of the CAR can comprise a CD3ζ chain portion and a co-stimulatory signaling region (domain). The co-stimulatory signaling region refers to a part of the CAR comprising the intracellular domain of a co-stimulatory molecule. A co-stimulatory molecule is a cell surface molecule other than an antigen receptor or their ligands that is required for an efficient response of lymphocytes to an antigen. Examples for a co-stimulatory molecule are CD27, CD28, 4-1BB (CD137), OX40, CD30, CD40, ICOS, lymphocyte function-associated antigen- 1 (LFA-1), CD2, CD7, LIGHT.

The cytoplasmic signaling sequences within the cytoplasmic signaling part of the CAR may be linked to each other with or without a linker in a random or specified order. A short oligo- or polypeptide linker, which is preferably between 2 and 10 amino acids in length, may form the linkage. A prominent linker is the glycine-serine doublet.

As an example, the cytoplasmic domain may comprise the signaling domain of CD3ζ and the signaling domain of CD28. In another example the cytoplasmic domain may comprise the signaling domain of CD3ζ and the signaling domain of CD137. In a further example, the cytoplasmic domain may comprise the signaling domain of CD3ζ, the signaling domain of CD28, and the signaling domain of CD137.

As aforementioned either the extracellular part or the transmembrane domain or the cytoplasmic domain of a CAR may also comprise a heterodimerizing domain for the aim of splitting key signaling and antigen recognition modules of the CAR.

The CAR may be further modified to include on the level of the nucleic acid encoding the CAR one or more operative elements to eliminate CAR expressing immune cells by virtue of a suicide switch. The suicide switch can include, for example, an apoptosis inducing signaling cascade or a drug that induces cell death. In one embodiment, the nucleic acid expressing and encoding the CAR can be further modified to express an enzyme such thymidine kinase (TK) or cytosine deaminase (CD). The CAR may also be part of a gene expression system that allows controlled expression of the CAR in the immune cell. Such a gene expression system may be an inducible gene expression system and wherein when an induction agent is administered to a cell being transduced with said inducible gene expression system, the gene expression system is induced and said CAR is expressed on the surface of said transduced cell.

In some embodiment the CAR may be a "SUPRA" (split, universal, and programmable) CAR, where a "zipCAR" domain may link an intra-cellular costimulatory domain and an extracellular leucine zipper (WO2017/091546). This zipper may be targeted with a complementary zipper fused e.g. to an scFv region to render the SUPRA CAR T cell tumor specific. This approach would be particularly useful for generating universal CAR T cells for various tumors; adapter molecules could be designed for tumor specificity and would provide options for altering specificity post-adoptive transfer, key for situations of selection pressure and antigen escape.

The CARs may be designed to comprise any portion or part of the above-mentioned domains as described herein in any order and/or combination resulting in a functional CAR, i.e. a CAR that mediated an immune effector response of the immune effector cell that expresses the CAR.

As used herein, the term "antigen" is intended to include substances that bind to or evoke the production of one or more antibodies and may comprise, but is not limited to, proteins, peptides, polypeptides, oligopeptides, lipids, carbohydrates such as dextran, haptens and combinations thereof, for example a glycosylated protein or a glycolipid. The term "antigen" as used herein refers to a molecular entity that may be expressed on the surface of a target cell and that can be recognized by means of the adaptive immune system including but not restricted to antibodies or TCRs, or engineered molecules including but not restricted to endogenous or transgenic TCRs, CARs, scFvs or multimers thereof, Fab-fragments or multimers thereof, antibodies or multimers thereof, single chain antibodies or multimers thereof, or any other molecule that can execute binding to a structure with high affinity.

The term "epitope" means the part of an antigen, e.g. a soluble antigen, that may be recognized and specifically bound by antibodies or antigen bindings fragments thereof (antigen binding domains).

The tumor associated antigen (TAA) as used herein refers to an antigenic substance produced in tumor cells. Tumor associated antigens are useful tumor or cancer markers in identifying tumor/cancer cells with diagnostic tests and are potential candidates for use in cancer therapy. Preferentially, the TAA may be expressed on the cell surface of the tumor/cancer cell, so that it may be recognized by the antigen binding receptor as disclosed herein.

The term "target cell" as used herein refers to cell which expresses an antigen on its cell surface that should be recognized (bound) by the antigen binding domain of conjugates as disclosed herein.

Said target cell may be e.g. a cancerous cell or a cell associated with an autoimmune disease or a cell associated with an infectious disease.

The terms "immune cell" or "immune effector cell" refer to a cell that may be part of the immune system and executes a particular effector function such as alpha-beta T cells, NK cells, NKT cells, B cells, innate lymphoid cells (ILC), cytokine induced killer (CIK) cells, lymphokine activated killer (LAK) cells, gamma-delta T cells, mesenchymal stem cells or mesenchymal stromal cells (MSC), monocytes or macrophages. Preferentially these immune cells are human immune cells. Preferred immune cells are cells with cytotoxic effector function such as alpha-beta T cells, NK cells, NKT cells, ILC, CIK cells, LAK cells or gamma-delta T cells. Most preferred immune effector cells are T cells and NK cells. "Effector function" means a specialized function of a cell, e.g. in a T cell an effector function may be cytolytic activity or helper activity including the secretion of cytokines.

### Examples

The following examples are intended for a more detailed explanation of the invention but without restricting the invention to these examples.

Example 1: Identification and selection of conjugates comprising an antigen recognizing moiety and a fluorescent dye via comparison with a reference clone allowing to discriminate between specific and non-specific binding on cell lines.

A series of novel antigen-binding fragments were derived from a phage library and expressed as single chain variable fragment-fragment crystallizable (scFv-Fc) fusion proteins. Subsequently, these scFv-Fc fusion proteins were coupled to Phycoerythrin (PE). In addition, Fab molecules against the same antigen were expressed and coupled to PE.

The labelled scFv-Fc fusion proteins and Fabs, respectively, were employed in cyclic immunofluorescence microscopy using the MACSima system which enables multiplexing. These labelled scFv-Fc fusion proteins and Fabs, respectively, were used to stain target antigen-expressing cells on one sample as well as target antigen-non-expressing cells on another sample and were compared to a reference clone targeting the same antigen.

First, PE-coupled scFv-Fc fusion proteins were detected on cells without expression of antigen of interest (Figure 2 A and B) and on cells with a transgenic knock-in of the antigen of interest (Figure 2 C and D). In a second step, binding of PE-coupled scFv-Fc fusion proteins was detected on cells with physiological expression, i.e. without genetic modification, of the antigen of interest (Figure 2 E and F). In a complementary approach binding of PE-coupled scFv-Fc fusion proteins was detected on cells with genetic ablation of the antigen of interest (Figure 2 G and H). PE-labelled scFv-Fc fusion proteins bound exclusively to antigen-expressing cells, but not to antigen-deficient cells (Figure 2 E and G). Image segmentation allowed to identify individual cells, i.e. units, from the sample, i.e. the array. Moreover, signal intensities of the candidate binders as well as positive and negative reference. The mean intensity value of the negative reference was set as lower threshold value, i.e. intensity values higher than the mean intensity value of the negative reference are considered to be specific signals. Subsequent analysis of single cells for co-expression of candidate binder and positive reference allows the identification of target-specific signal of the binder candidate. Here, the candidates have a similar staining pattern as the reference antibodies in terms of intensity as well as distribution (Figure 2 B, D, F, H) indicating antigen-specificity.

In another approach Fab signal was detected as direct conjugate (Fab-PE) on cells with a transgenic knock-in of the antigen of interest (Figure 3 A and C). Subsequently, Fabs were detected as direct conjugate (Fab-PE) on cells with physiological expression of the target antigen and on cells with a CRISPR/Cas9-mediated knock-out of the antigen of interest (Figure 3 E and G). Image segmentation allowed to identify individual cells, i.e. units, from the sample, i.e. the array. Moreover, signal intensities of the candidate binders as well as positive and negative reference. The mean intensity value of the negative reference was set as lower threshold value, i.e. intensity values higher than the mean intensity value of the negative reference are considered to be specific signals. Subsequent analysis of single cells for co-expression of candidate binder and positive reference allows the identification of target-specific signal of the binder candidate. Here, the candidates have a similar staining pattern as the reference antibodies in terms of intensity as well as distribution indicating antigen-specificity. Labelled Fab molecules bound exclusively to antigen-expressing cells, but not to antigen-non-expressing cells. Moreover, they have a similar staining pattern as the reference antibody in terms of intensity as well as distribution (Figure 3 B, D, F, H.).

Example 2: Identification and selection of conjugates comprising an antigen recognizing moiety and a fluorescent dye via comparison with a reference clone allowing to discriminate between specific and non-specific binding on human tissue.

First, scFv-Fc fusion proteins were detected on tumor tissue which is composed of target cells as well as non-target cells (Figure 4 A). PE-labelled scFv-Fc fusion proteins bound exclusively to target cells, but not to non-target cells as indicated by a comparable staining profile as the reference antibody or a non-target cell marker, respectively. Moreover, they have a similar staining pattern as the reference antibody in terms of intensity as well as distribution (Figure 4 B).

Fabs were detected as direct conjugate (Fab-PE) on tumor tissue which is composed of target cells as well as non-target cells. PE-labelled Fabs bound exclusively to target cells, but not to non-target cells (Figure 5 A) as indicated by a comparable staining profile as the reference antibody or a non-target cell binding marker, respectively. Image segmentation allowed to identify individual cells, i.e. units, from the sample, i.e. the array. Moreover, signal intensities of the candidate binders as well as positive and negative reference. The mean intensity value of the negative reference was set as lower threshold value, i.e. intensity values higher than the mean intensity value of the negative reference are considered to be specific signals. Subsequent analysis of single cells for co-expression of candidate binder and positive reference allows the identification of target-specific signal of the binder candidate. Here, the candidates have a similar staining pattern as the reference antibodies in terms of intensity as well as distribution indicating antigen-specificity. Moreover, they have a similar staining pattern as the reference antibody in terms of intensity as well as distribution (Figure 5 B).

Example 3: Identification and selection of conjugates comprising an antigen recognizing moiety and a fluorescent dye via comparison with a reference clone allowing to discriminate between specific and non-specific binding on human tissue micro arrays.

In order to analyze a panel of human tissues in a single experiment human tissue micro arrays were analyzed for target expression. PE-labelled Fabs and PE-labelled scFv-Fc fusion proteins detected on human tissue micro arrays which were composed of several healthy tissues. These tissues themselves are composed of target cells as well as non-target cells. Some PE-labelled Fabs and PE-labelled scFv-Fc fusion proteins bound exclusively to target cells, but not to non-target cells (Figure 6) as indicated by a comparable staining profile as the reference antibody or a non-target cell marker, respectively. Image segmentation allowed to identify individual cells, i.e. units, from the sample, i.e. the array. Moreover, signal intensities of the candidate binders as well as positive and negative reference. The mean intensity value of the negative reference was set as lower threshold value, i.e. intensity values higher than the mean intensity value of the negative reference are considered to be specific signals. Subsequent analysis of single cells for co-expression of candidate binder and positive reference allows the identification of target-specific signal of the binder candidate. Here, the candidates have a similar staining pattern as the reference antibodies in terms of intensity as well as distribution indicating antigen-specificity. Moreover, they have a similar staining pattern as the reference antibody in terms of intensity as well as distribution (Figure 6).

scFv-Fc candidates were detected as direct conjugate (scFv-Fc-PE) on healthy tissues (Figure 6 A breast, C lung, E brain) which are composed of target cells and non-target cells or of non-target cells only. PE-labelled scFv-Fc bound exclusively to target cells, but not to non-target cells as indicated by a comparable staining profile as the reference antibody or a non-target cell binding marker, respectively. Moreover, they have a similar staining pattern as the reference antibody in terms of intensity as well as distribution (Figure 6 B breast, D lung, F brain).

Fabs were detected as direct conjugate (Fab-PE) on healthy tissues (Figure 6 G breast, I lung, K brain) which are composed of target cells and non-target cells or of non-target cells only. PE-labelled Fabs bound exclusively to target cells, but not to non-target cells as indicated by a comparable staining profile as the reference antibody or a non-target cell binding marker, respectively. Moreover, they have a similar staining pattern as the reference antibody in terms of intensity as well as distribution (Figure 6 H breast, J lung, L brain).

### References

Tan WCC, Nerurkar SN, Cai HY, Ng HHM, Wu D, Wee YTF, Lim JCT, Yeong J, Lim TKH. Overview of multiplex immunohistochemistry/immunofluorescence techniques in the era of cancer immunotherapy. Cancer Commun (Lond). 2020 Apr;40(4): 135-153. doi: 10.1002/cac2.12023. Epub 2020 Apr 17. PMID: 32301585; PMCID: PMC7170662.

## Claims

1. An *in-vitro* method for selecting antigen recognizing moieties having specificity for a target structure, the method comprising
a) providing an aggregate comprising spatially separated units comprising
i) first units that present said target structure, or
ii) first units that present said target structure and second units that do not present said target structure
b) contacting said aggregate with a first conjugate comprising a fluorescent moiety and a first antigen recognizing moiety that can bind to said target structure,
c) removing unbound first conjugate from said aggregate of step b) and determining
i) the intensity of the fluorescence signal emitted by the fluorescent moieties of the first conjugate present in/on each unit of said aggregate, and
ii) the spatial distribution of the intensities of the fluorescence signals emitted by the fluorescent moieties of the first conjugate present in/on the units of said aggregate, thereby identifying a pattern reflecting the presence of said first conjugate in/on units of said aggregate,
d) repeating steps b) and c) at least once by erasing the preceding fluorescence signal emitted by the fluorescent moieties of the preceding conjugate with at least one further conjugate comprising a fluorescent moiety and a further antigen recognizing moiety that can bind to said target structure, wherein said first conjugate or one of said at least one further conjugates is a positive reference
e) identifying and selecting from the conjugates of step b) to d), that are not the positive reference, the conjugates with antigen recognizing moieties that have an at least 45% overlap of the pattern as determined in step c) ii) relative to the pattern of the positive reference.

2. The method of claim 1, wherein said target structure is a target antigen expressed on the surface of a target cell,
and wherein said first units of said aggregate comprise individual units comprising
a) target cells expressing said target antigen on the surface of said target cells, or
b) cells expressing recombinantly said target antigen on the surface of said cells, wherein said cells expressing recombinantly said target antigen are a positive control;
and optionally in addition to a) or b):
c) non-target cells or tissue that express said target antigen on the surface of said non-target cells or of the cells of said tissue;
and wherein said second units of said aggregate comprise individual units comprising non-target cells or tissue that do not present said target antigen on the surface of said non target cells or of the cells of said tissue, wherein each individual unit comprise only one type of non-target cells or one type of tissue.

3. The method of claim 2, wherein said second units of said aggregate comprise additionally an individual unit comprising cells that are recombinantly knocked-out for said target antigen as a negative control.

4. The method of claim 2 or 3, wherein said second units of said array comprise at least two individual units having different types of non-target cells or type of tissues.

5. The method of claim 1 to 4, wherein said aggregate is a cluster of first and second units on one solid phase or said aggregate is collection of first and second units that are all or partly on different solid phases allowing also a timely separated processing of the units of the aggregate that are not on the same solid phase.

6. The method of claim 2 to 5, wherein said target cells expressing said target antigen on the surface of said target cells are cancer cells or tumor tissue, and wherein said non-target cells or tissue that express said target antigen on the surface of said non-target cells or of the cells of said tissue is/are healthy tissue or cancer cells of another type of cancer than the cancer cells/tumor tissue that are the target cells, and wherein said target antigen is a tumor associated antigen (TAA).

7. The method of claim 2 to 6, wherein said an in-vitro method additionally comprises the step of providing an immune cell with a chimeric antigen receptor (CAR) comprising
I) an antigen recognizing moiety
II) a transmembrane domain
III) an intracellular signaling domain,
wherein the antigen recognizing moiety of the CAR is an antigen recognizing moiety identified and selected in step e).

8. An immune cell expressing a CAR comprising
I) an antigen recognizing moiety
II) a transmembrane domain, and
III) an intracellular signaling domain,
wherein the antigen recognizing moiety of the CAR is obtained by the method according to claim 2 to 6.
